# EUROPÄISCHE PATENTSCHRIFT

(11) **EP 1 078 004 B1**
(45) Veröffentlichungstag und Bekanntmachung des Hinweises auf die Patenterteilung: **31.10.2007**
(21) Anmeldenummer: 99932626.7
(22) Anmeldetag: 05.05.1999
(51) Int. Cl.: C07K 16/00, C07K 16/28, C12N 15/63, A61K 39/395, G01N 33/53

(54) **Tetravalente Antikörperkonstrukte**
Tetravalent antibody constructs
Constructions d'anticorps tetravalents

(30) Priorität: 05.05.1998 DE 19819846
(43) Veröffentlichungstag der Anmeldung: 28.02.2001
(73) Patentinhaber: Deutsches Krebsforschungszentrum Stiftung des öffentlichen Rechts, 69120 Heidelberg (DE)
(72) Erfinder: LITTLE, Melvyn, D-69151 Neckargemünd (DE); KIPRIYANOV, Sergej, D-69121 Heidelberg (DE)
(74) Vertreter: Schüssler, Andrea
(86) Internationale Anmeldenummer: PCT/DE1999/001350
(87) Internationale Veröffentlichungsnummer: WO 1999/057150

(56) Entgegenhaltungen:
- EP-A- 0 952 218
- GRUBER M ET AL: "Efficient tumor cell lysis mediated by a bispecific single chain antibody expressed in Escherichia coli." JOURNAL OF IMMUNOLOGY, (1994 JUN 1) 152 (11) 5368-74. , XP000872832
- MACK M ET AL: "A small bispecific antibody construct expressed as a functional single-chain molecule with high tumor cell cytotoxicity." PROCEEDINGS OF THE NATIONAL ACADEMY OF SCIENCES OF THE UNITED STATES OF AMERICA, (1995 JUL 18) 92 (15) 7021-5. , XP000566333
- KURUCZ I ET AL: "Retargeting of CTL by an efficiently refolded bispecific single-chain Fv dimer produced in bacteria." JOURNAL OF IMMUNOLOGY, (1995 MAY 1) 154 (9) 4576-82. , XP000872833
- DE JONGE J ET AL: "Production and characterization of bispecific single-chain antibody fragments." MOLECULAR IMMUNOLOGY, (1995 DEC) 32 (17-18) 1405-12. , XP000872314
- COLOMA M J ET AL: "Design and production of novel tetravalent bispecific antibodie [see comments]." NATURE BIOTECHNOLOGY, (1997 FEB) 15 (2) 159-63. , XP000647731
- KIPRIYANOV S M ET AL: "Bispecific tandem diabody for tumor therapy with improved antigen binding and pharmacokinetics." JOURNAL OF MOLECULAR BIOLOGY, (1999 OCT 15) 293 (1) 41-56. , XP002131382

## Beschreibung

Die vorliegende Erfindung betrifft multivalente Fᵥ-Antikörper-Konstrukte, sie kodierende Expressionsplasmide, und ein Verfahren zur Herstellung der Fᵥ-Antikörper-Konstrukte sowie ihre Verwendung.

Natürliche Antikörper sind Dimere und werden daher als bivalent bezeichnet. Sie weisen vier variable Domänen, nämlich zwei V_{H}- und zwei V_{L}-Domänen, auf. Die variablen Domänen dienen als Bindungsstellen für ein Antigen, wobei eine Bindungsstelle aus einer V_{H}- und einer V_{L}-Domäne ausgebildet ist. Natürliche Antikörper erkennen jeweils ein Antigen, wodurch sie auch als monospezifisch bezeichnet werden. Ferner weisen sie auch konstante Domänen auf. Diese tragen zur Stabilität der natürlichen Antikörper bei. Andererseits sind sie auch für unerwünschte Immunreaktionen mitverantwortlich, die entstehen, wenn natürliche Antikörper verschiedener Tierarten wechselseitig verabreicht werden.

Zur Vermeidung solcher Immunreaktionen werden Antikörper konstruiert, denen die konstanten Domänen fehlen. Insbesondere sind dies Antikörper, die nur noch die variablen Domänen aufweisen. Solche Antikörper werden mit Fᵥ-Antikörper-Konstruken bezeichnet. Diese liegen häufig in Form einzelkettiger, sich miteinander gepaarter Monomere vor.

Es hat sich allerdings gezeigt, daß Fᵥ-Antikörper-Konstrukte nur eine geringe Stabilität aufweisen. Ihre Verwendbarkeit für therapeutische Zwecke ist daher stark eingeschränkt.

Im Stand der Technik sind Bispezifische single-chain Fᵥ-Antikörpermoleküle mit vier variablen Domänen bekannt, die über Peptidlinker miteinander verbunden sind (Journal of Immunology, 152(11), (1994), 5368-74; Proceedings of the National Acadamy of the United States of America, 92(15), (1995), 7021-5; Journal of Immunology, 154(9), (1995), 4576-82 und Molecular Immunology, 32(17-18), (1995), 1405-12).

In der EP 0952218 wird ein multivalentes Antikörperkonstrukt in der Form von V_{H}-L-V_{L}P-V_{H}-L-V_{L} beschrieben, wobei mit V variable Domänen und mit L sowie P Peptidlinker bezeichnet werden. Die Linker L sind 1-20 Aminosäuren lang und der Linker P ist 12-20 Aminosäuren lang. Durch den 12-20 Aminosäuren langen Linker P faltet sich das Konstrukt intramolekular.

Der vorliegenden Erfindung liegt somit die Aufgabe zugrunde, einen Antikörper bereitzustellen, mit dem unerwünschte Immunreaktionen vermieden werden können. Ferner soll er eine Stabilität aufweisen, die ihn für therapeutische Zwecke einsetzbar macht. Erfindungsgemäß wird dies durch die Gegenstände in den Patentansprüchen erreicht.

Gegenstand der vorliegenden Erfindung ist somit ein tetravalenter Fᵥ-Antikörper-Diner Konstrukt, der eine große Stabilität aufweist. Ein solches eignet sich für diagnostische und therapeutische Zwecke.

Die vorliegende Erfindung beruht auf den Erkenntnissen des Anmelders, daß die Stabilität eines Fᵥ-Antikörper-Konstruktes erhöht werden kann, wenn dieses in Form eines einzelkettigen Dimeres vorliegt, bei dem die vier variablen Domänen über drei Peptidlinker miteinander verbunden sind. Ferner hat der Anmelder erkannt, daß sich das Fᵥ-Antikörper-Konstrukt mit sich selbst faltet, wenn der mittlere Peptidlinker eine Länge von etwa 10 - 30 Aminosäuren aufweist. Des weiteren hat der Anmelder erkannt, daß sich das Fᵥ-Antikörper-Konstrukt mit anderen Fᵥ-Antikörper-Konstrukten zusammenfaltet, wenn der mittlere Peptidlinker eine Länge von etwa bis zu 10 Aminosäuren aufweist, wodurch ein multimeres, d.h. multivalentes, Fᵥ-Antikörper-Konstrukt erhalten wird. Auch hat der Anmelder erkannt, daß das Fᵥ-Antikörper-Konstrukt multispezifisch sein kann.

Erfindungsgemäß werden die Erkenntnisse des Anmelders genutzt, ein multivalentes Fᵥ-Antikörper-Konstrukt nach Anspruch 1 bereitzustellen, das vier variable Domänen umfaßt, die über die Peptidlinker 1, 2 und 3 miteinander verbunden sind.

Der Ausdruck "Fᵥ-Antikörper"- weist auf einen Antikörper hin, der variable Domänen, nicht aber konstante Domänen aufweist.

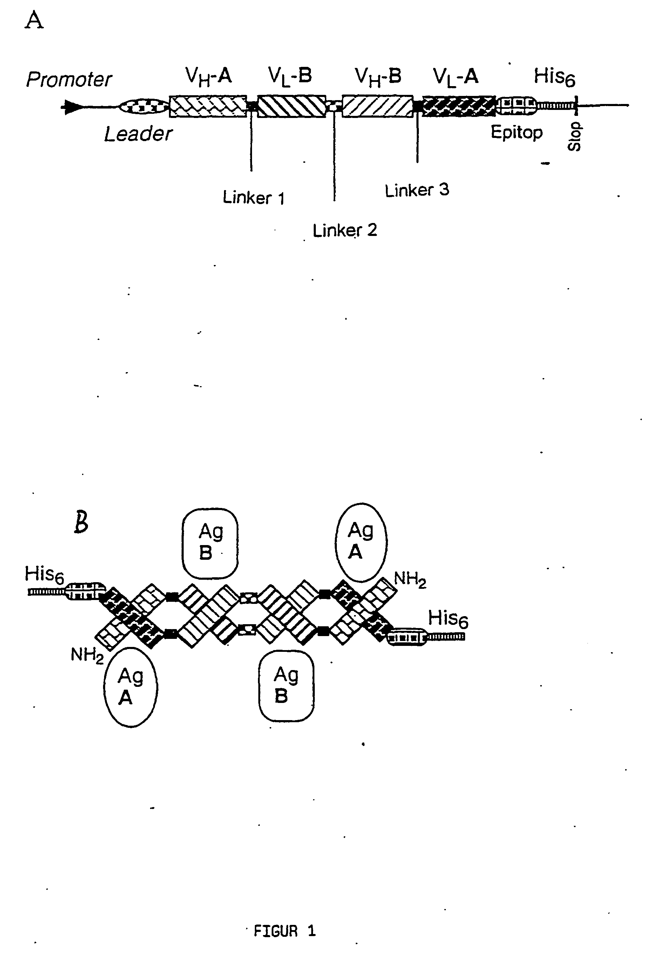

Der Ausdruck "multivalentes Fᵥ-Antikörper-Konstrukt" weist auf einen Fᵥ-Antikörper hin, der mehrere variable Domänen, jedoch mindestens vier aufweist. Solches wird erreicht, wenn sich das einzelkettige Fᵥ-Antikörper-Konstrukt mit anderen einzelkettigen Fᵥ-Antikörper-Konstrukten zusammenfaltet. In diesem Fall liegt ein Fᵥ-Antikörper-Konstrukt vor, das 8, variable Domänen aufweist. Günstig ist es, wenn das Fᵥ-Antikörper-Konstrukt vier variable Domänen aufweist, d.h. oder tetravalent (vgl. Fig. 1). Ferner können die variablen Domänen gleich oder verschieden voneinander sein, wodurch das Antikörper-Konstrukt ein oder mehrere Antigene erkennt. Vorzugsweise erkennt das Antikörper-Konstrukt ein oder zwei Antigene, d.h. es ist mono- bzw. bispezifisch. Beispiele solcher Antigene sind die Proteine CD19 und CD3.

Der Ausdruck "Peptidlinker 1, 3" weist auf einen Peptidlinker hin, der geeignet ist, variable Domänen eines Fᵥ-Antikörper-Konstruktes miteinander zu verbinden. Der Peptidlinker kann jegliche Aminosäuren enthalten, wobei die Aminosäuren Glycin (G), Serin (S) und Prolin (P) bevorzugt sind. Die Peptidlinker 1 und 3 können gleich oder verschieden voneinander sein. Ferner kann der Peptidlinker eine Länge von etwa 0 - 10 Aminosäuren aufweisen. In ersterem Fall ist der Peptidlinker lediglich eine Peptidbindung aus dem COOH-Rest einer der variablen Domänen und dem NH₂-Rest einer anderen der variablen Domänen. Vorzugsweise weist der Peptidlinker die Aminosäuresequenz GG auf.

Der Ausdruck "Peptidlinker 2" weist auf einen Peptidlinker hin, der geeignet ist, variable Domänen eines Fᵥ-Antikörper-Konstruktes miteinander zu verbinden. Der Peptidlinker kann jegliche Aminosäuren enthalten, wobei die Aminosäuren Glycin (G), Serin (S) und Prolin (P) bevorzugt sind. Ferner kann der Peptidlinker eine Länge von etwa 3-10 Aminosäuren, insbesondere 5 Aminosäuren, und ganz besonders die Aminosäuresequenz GGPGS, aufweisen, wodurch erreicht wird, daß sich das einzelkettige Fᵥ-Antikörper-Konstrukt mit anderen einzelkettigen Fᵥ-Antikörper-Konstrukten zusammenfaltet.

Ein erfindungsgemäße tetravalenter Fᵥ-Antikörper-Dimer kann durch übliche Verfahren hergestellt werden. Günstig ist ein Verfahren, bei dem für die Peptidlinker 1, 2 und 3 kodierende DNAs mit für die vier variablen Domänen eines Fᵥ-Antikörper-Konstruktes kodierenden DNAs ligiert werden derart, daß die Peptidlinker die variablen Domänen miteinander verbinden, und das erhaltene DNA-Molekül in einem Expressionsplasmid exprimiert wird. Es wird auf die Beispiele 1 - 6 verwiesen. Hinsichtlich der Ausdrücke "Fᵥ-Antikörper-Konstrukt" und "Peptidlinker" wird auf vorstehende Ausführungen verwiesen. Ergänzend wird auf Maniatis, T. et al., Molecular Cloning, A Laboratory Manual, Cold Spring Harbor Laboratory 1982, verwiesen.

DNAs, die für ein erfindungsgemäßes Fᵥ-Antikörper-Konstrukt kodieren, sind ebenfalls Gegenstand der vorliegenden Erfindung. Ferner sind Expressionsplasmide, die solche DNAs enthalten, auch Gegenstand der vorliegenden Erfindung. Bevorzugte Expressionsplasmide sind pDISC3x19-SL, pPIC-DISC-SL, und pDISC6-SL. Die ersteren zwei wurden bei der DSMZ (Deutsche Sammlung für Mikroorganismen und Zellen) am 30. April 1998 unter DSM 12149, bzw. DSM 12151 hinterlegt.

Ein weiterer Gegenstand der vorliegenden Erfindung ist ein Kit, umfassend:
(a) ein erfindungsgemäße tetravalentes Fᵥ-Antikörper-dimer und/oder
(b) ein erfindungsgemäßes Expressionsplasmid, sowie
(c) übliche Hilfsstoffe, wie Puffer, Lösungsmittel und Kontrollen.

Von den einzelnen Komponenten können ein oder mehrere Vertreter vorliegen.

Die vorliegende Erfindung stellt-ein multivalentes tetravalentes Fᵥ-Antikörper- Dimer bereit, bei dem die variablen Domänen über Peptidlinker miteinander verbunden sind. Ein solches Antikörper-Konstrukt zeichnet sich dadurch aus, daß es keine Teile enthält, die zu unerwünschten Immunreaktionen führen können. Ferner weist es eine große Stabilität auf. Des weiteren ermöglicht es mehrere Antigene gleichzeitig zu binden. Das erfindungsgemäße Fᵥ-Antikörper-Konstrukt eignet sich daher bestens nicht nur für diagnostische, sondern auch für therapeutische Zwecke verwendet zu werden. Solche Zwecke können hinsichtlich jeder Erkrankung, insbesondere einer viralen, bakteriellen oder Tumor-Erkrankung, gesehen werden.

### Kurze Beschreibung der Zeichnungen:

**Fig. 1** zeigt die genetische Organisation eines erfindungsgemäßen Fᵥ-Antikörper-Konstruktes (A) und Schemata zur Bildung eines (B) tetravalenten Fᵥ-Antikörper-Konstruktes Ag: Antigen; His₆: sechs C-terminale Histidinreste; Stop: Stoppcodon (TAA); V_{H} und V_{L}: variable Region der schweren und der leichten Kette.

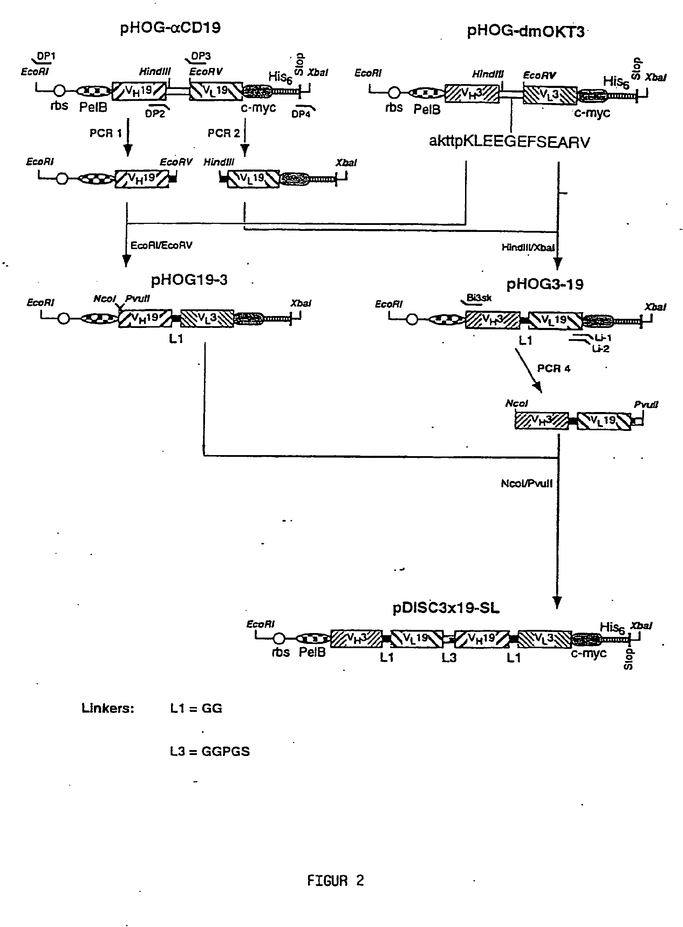

**Fig. 2** zeigt das Schema zur Konstruktion des Plasmids pDISC3x19-SL. c-myc: Sequenz, kodierend für ein Epitop, das von dem Antikörper 9E1 erkannt wird, His₆: Sequenz, die für sechs C-terminale Histidinreste kodiert; PeIB: Signalpeptidsequenz der bakteriellen Pectatlyase (PelB-Leader); rbs: Ribosomenbindungsstelle; Stop: Stoppcodon (TAA); V_{H} und V_{L}: variable Region der schweren und der leichten Kette.

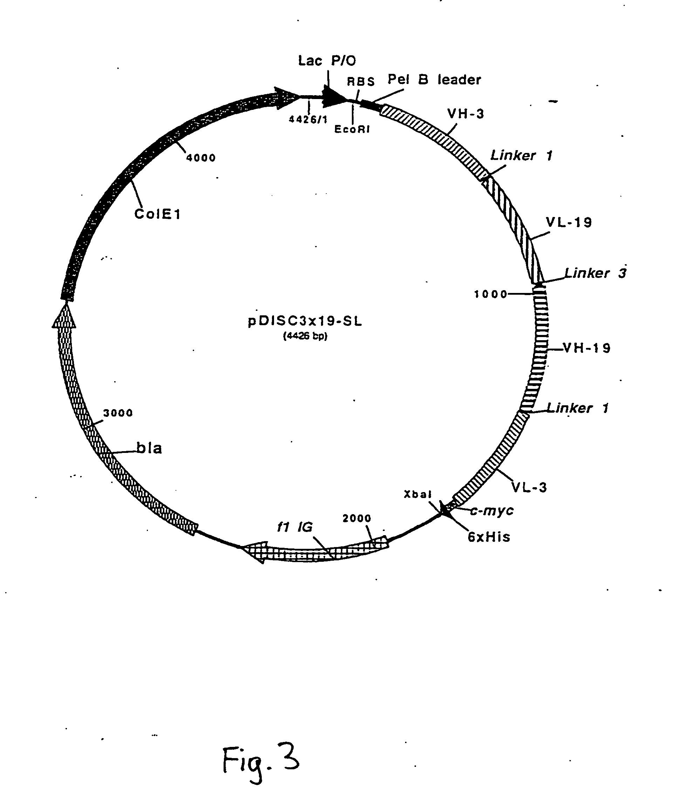

**Fig. 3** zeigt ein Diagramm des Expressionsplasmids pDISC3x19-SL. 6xHis: Sequenz, die für sechs C-terminale Histidinreste codiert; bla: Gen, das für β-Lactamase kodiert, die für Ampicillinresistenz verantwortlich ist; bp: Basenpaare; *c-myc:* Sequenz, kodierend für ein Epitop, das von dem Antikörper 9E10 erkannt wird; ColE1: Origin der DNA-Replikation; f1-IG: intergenische Region des Bakteriophagen f1; Lac P/O: wt *lac*-Operon-Promotor/Operator; Linker 1: Sequenz, die für ein GlyGly-Dipeptid codiert, das die V_{H}- und V_{L}-Domänen verknüpft; Linker 3: Sequenz, die für ein GlyGlyProGlySer-Oligopeptid codiert, das die hybriden scFv-Fragmente verknüpft; Pel-B-Leader: Signalpeptidsequenz der bakteriellen Pectatlyase; rbs: Ribosomenbindungsstelle; V_{H} und V_{L}: variable Region der schweren und der leichten Kette.

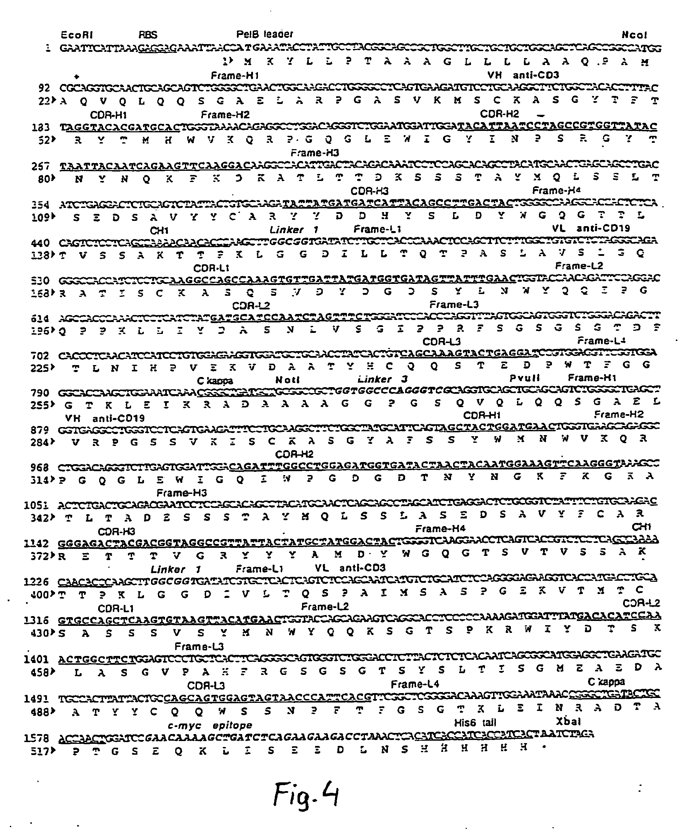

**Fig. 4** zeigt die Nukleotid- und die abgeleitete Aminosäuresequenz des durch das Expressionsplasmid pDISC3×19-SL kodierten tetravalenten Fᵥ-Antikörper-Konstruktes. *c-myc*-Epitop: Sequenz, kodierend für ein Epitop, das von dem Antikörper 9E10 erkannt wird; CDR: Komplementarität bestimmende Region, Gerüst: Gerüstregion (Framework-Region); His6-Schwanz, Sequenz, die für sechs C-terminale Histidinreste kodiert; PelB-Leader: Signalpeptidsequenz der bakteriellen Pectalyase; RBS: Ribosomenbindungsstelle; V_{H} und V_{L}: variable Region der schweren und der leichten Kette.

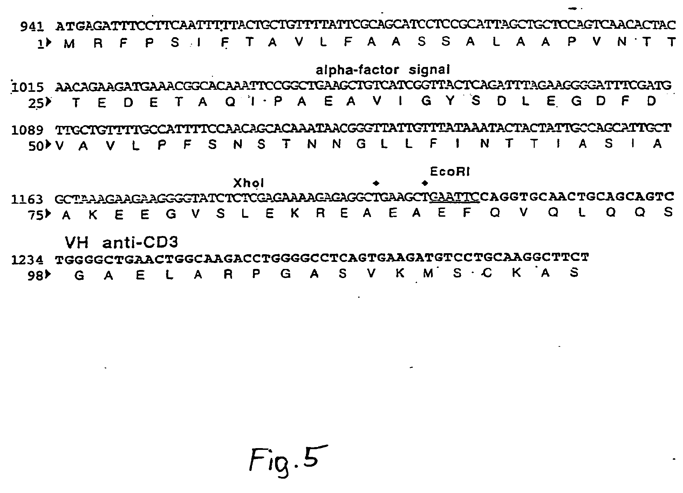

**Fig. 5** zeigt die Nukleotid- und die abgeleitete Aminosäuresequenz einer Verbindung zwischen einem Gen, das für eine α-Faktor-Leadersequenz kodiert, und einem Gen, das für das tetravalente Fᵥ-Antikörper-Konstrukt codiert, in dem Pichia-Expressionsplasmid pPIC-DISC-SL. Alpha-Faktor-Signal: Leaderpeptidsequenz des *Saccharomyces cerevisiae-α-*Faktor-Sekretionssignals; V_{H}: variable Region der schweren Kette. Rauten zeigen die Signalspaltstellen an.

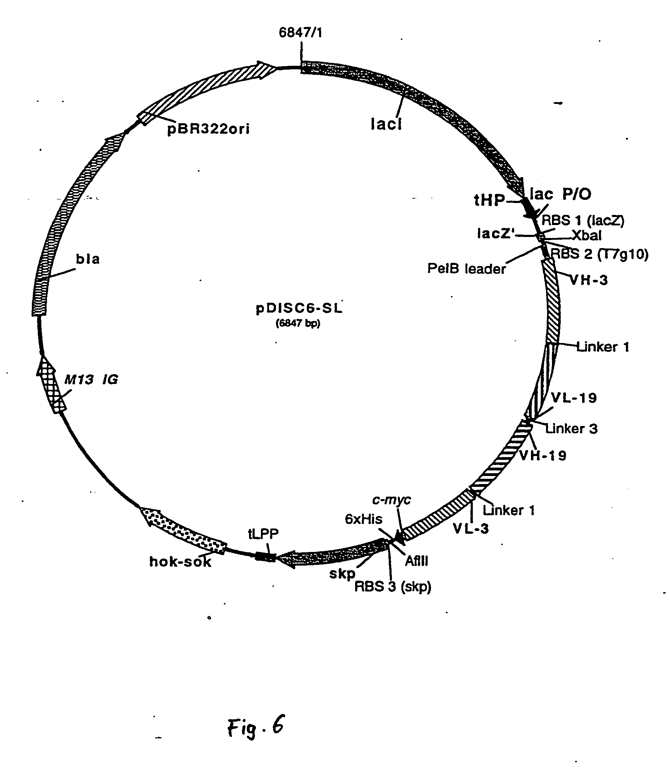

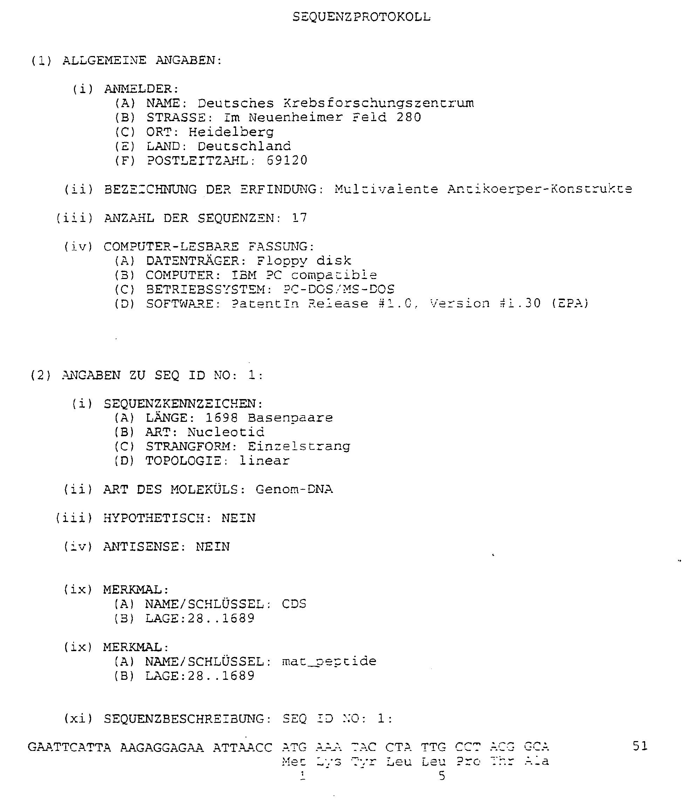

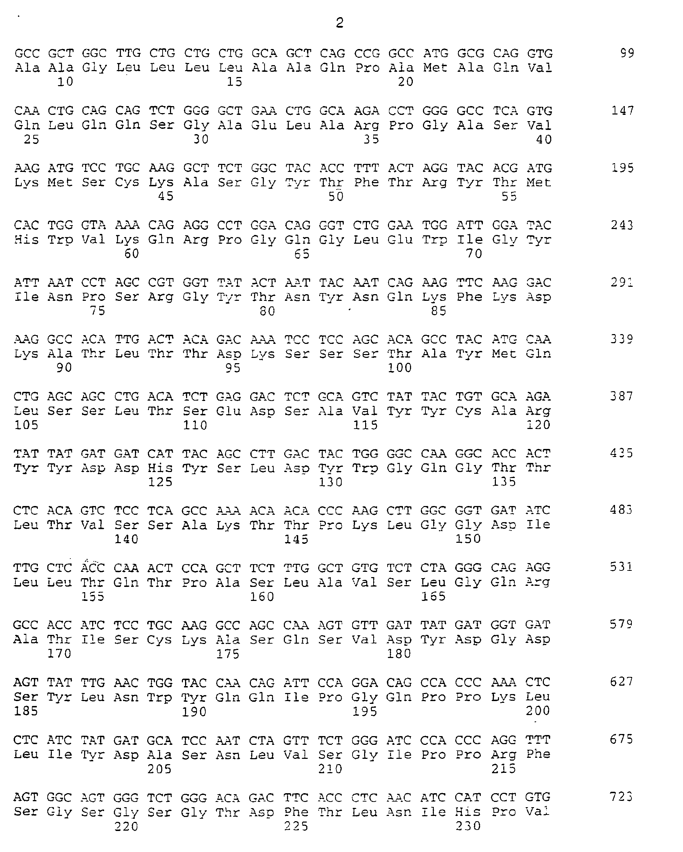

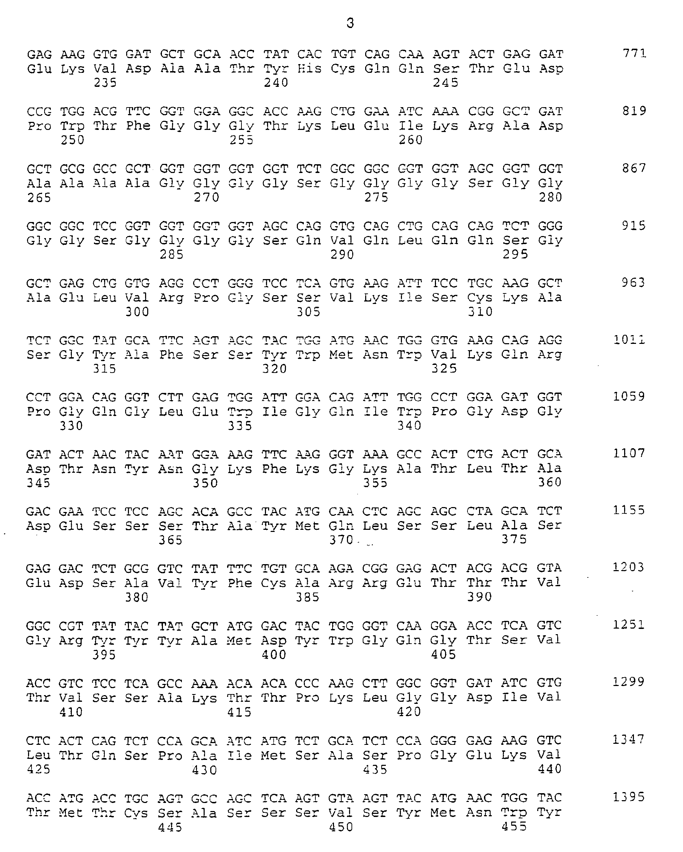

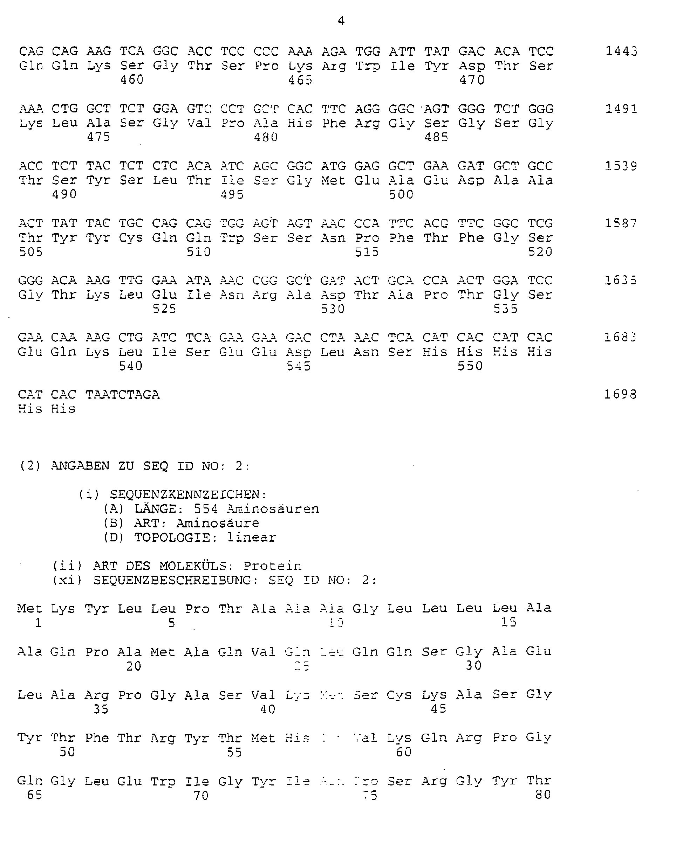

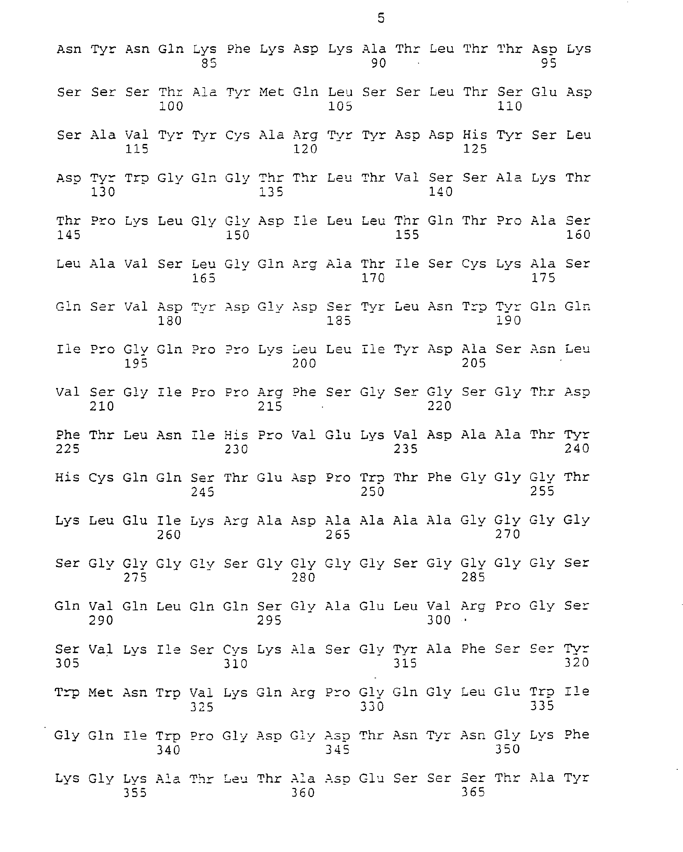

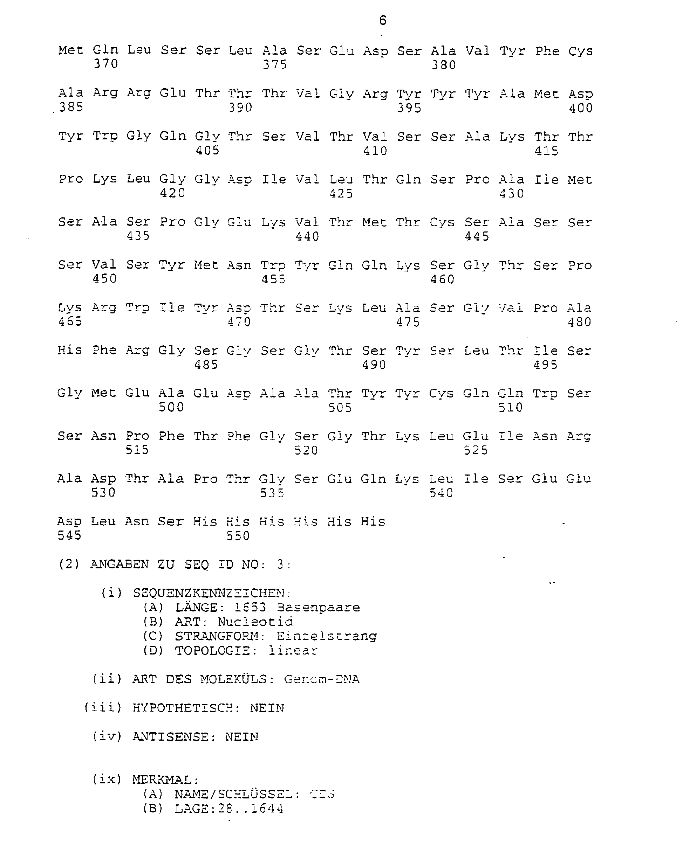

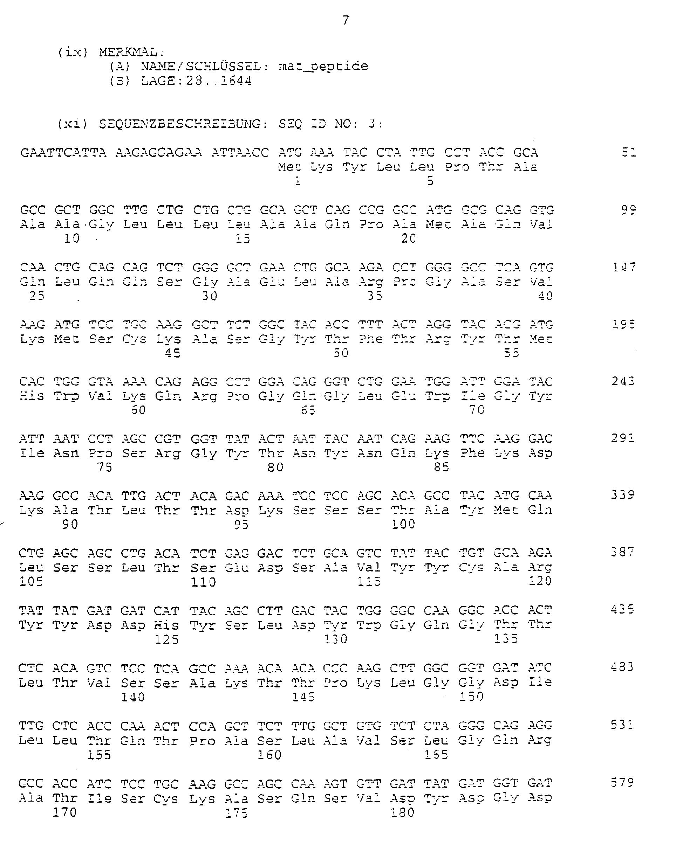

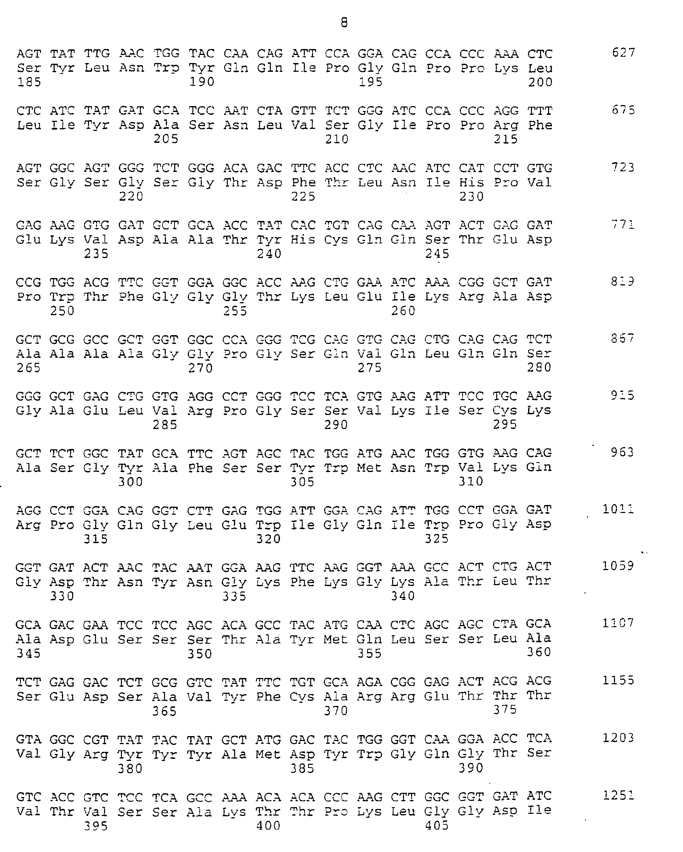

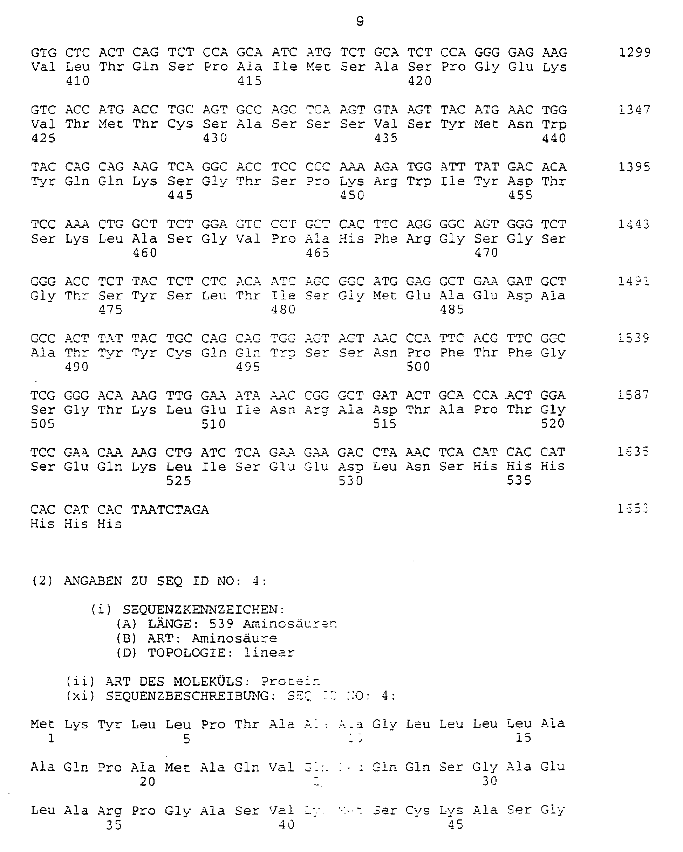

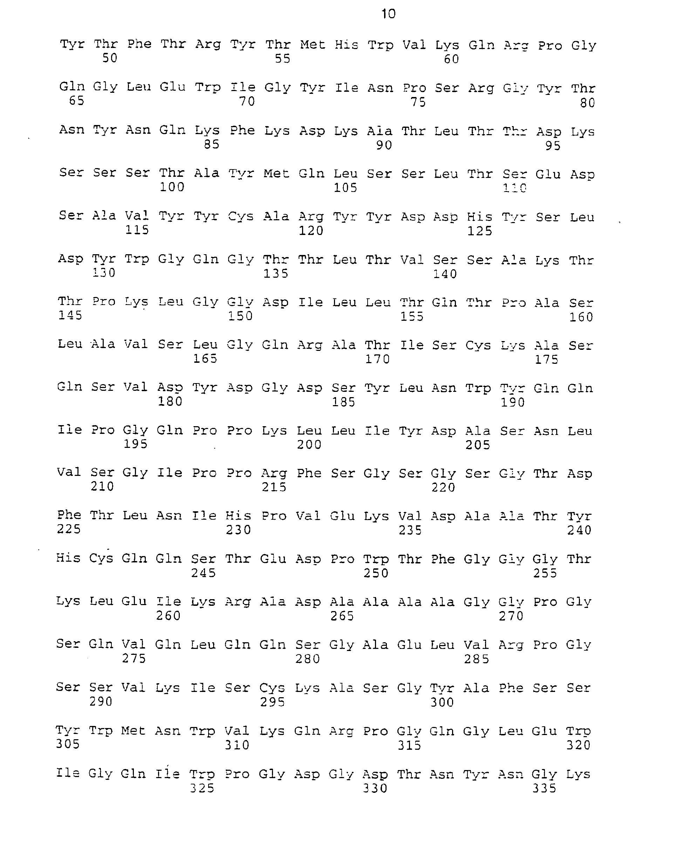

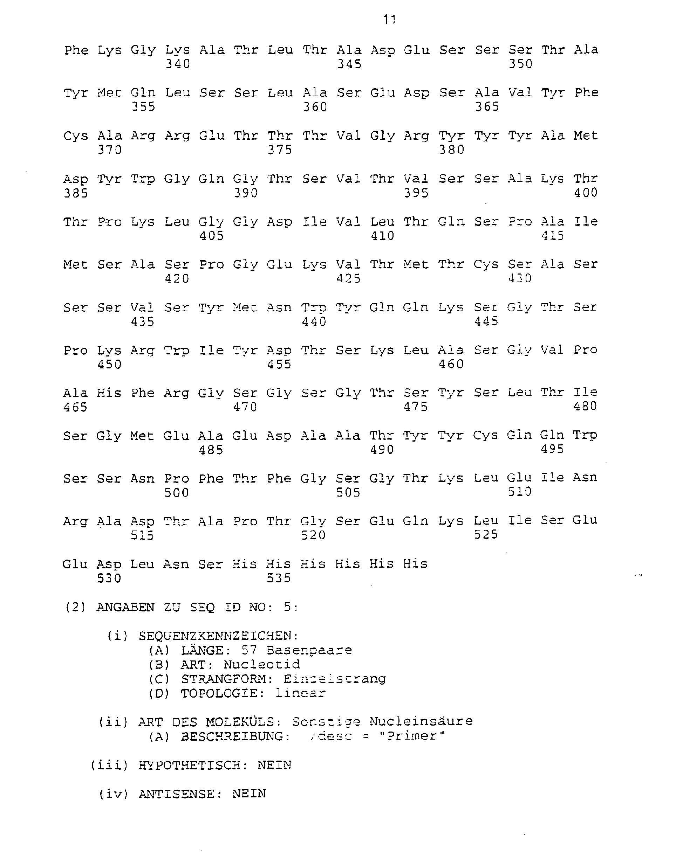

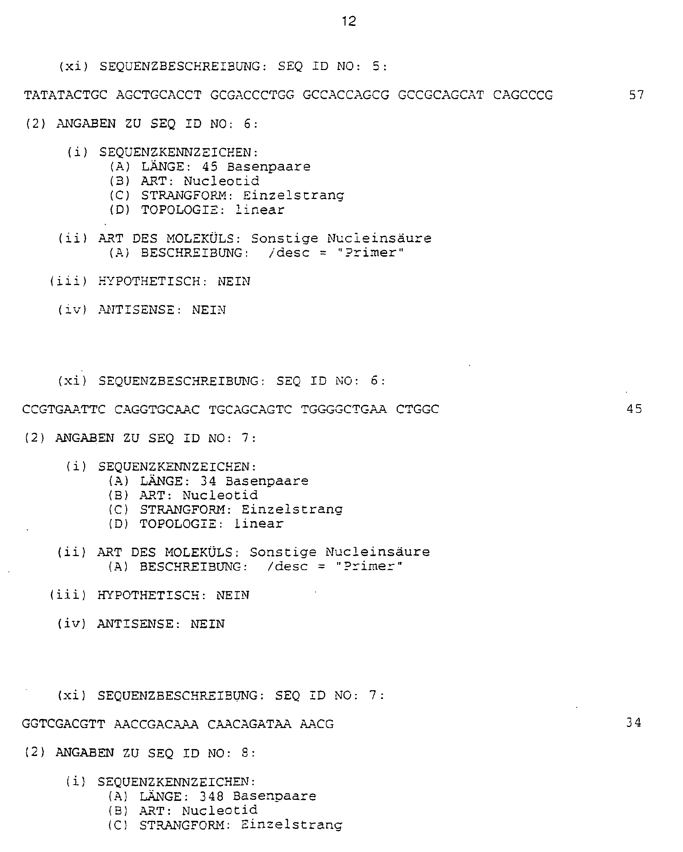

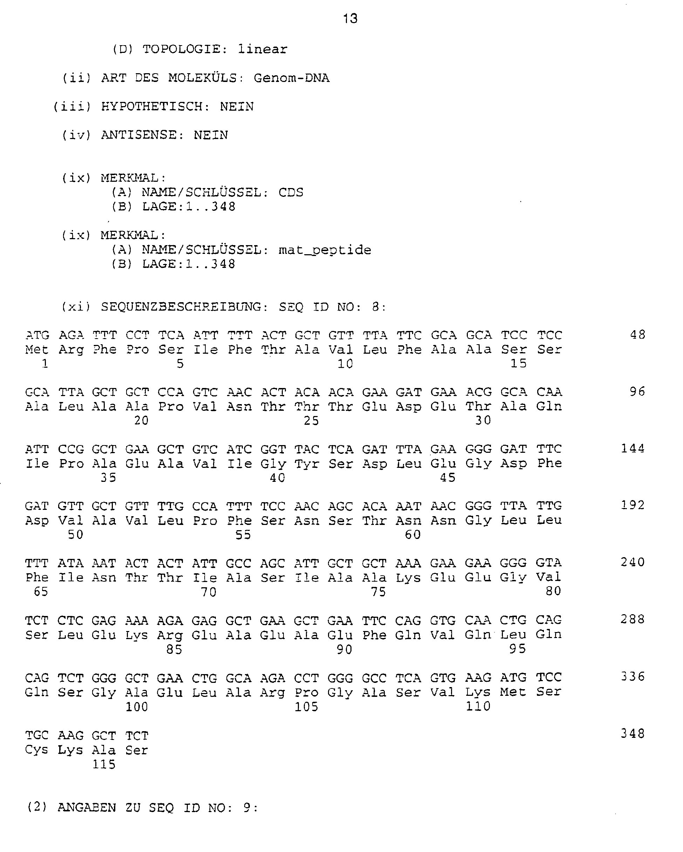

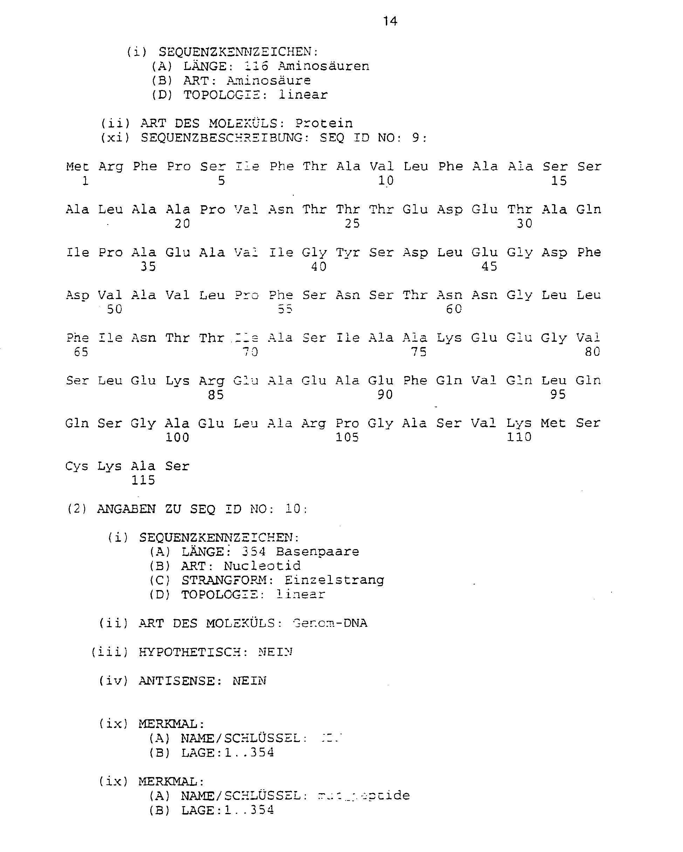

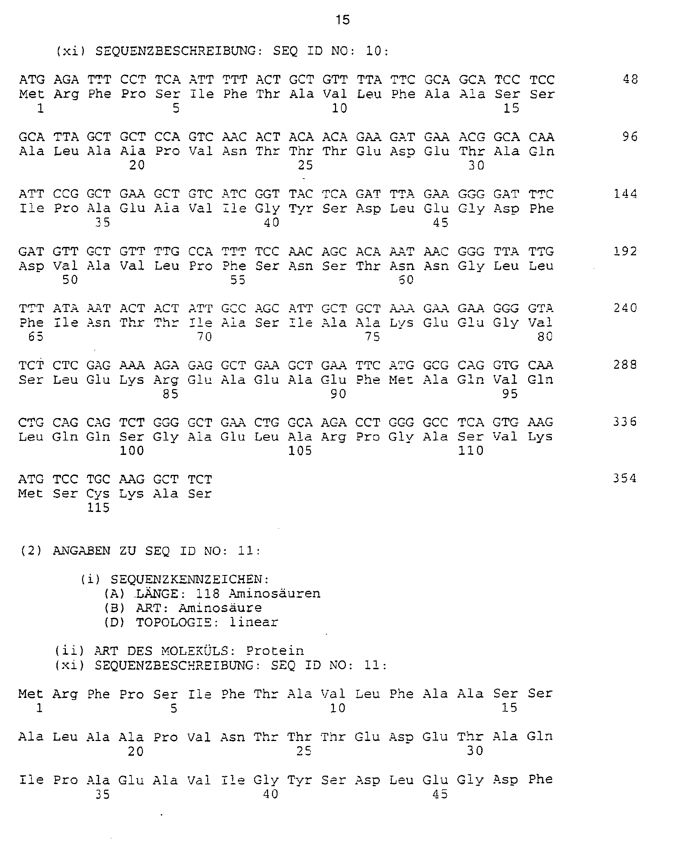

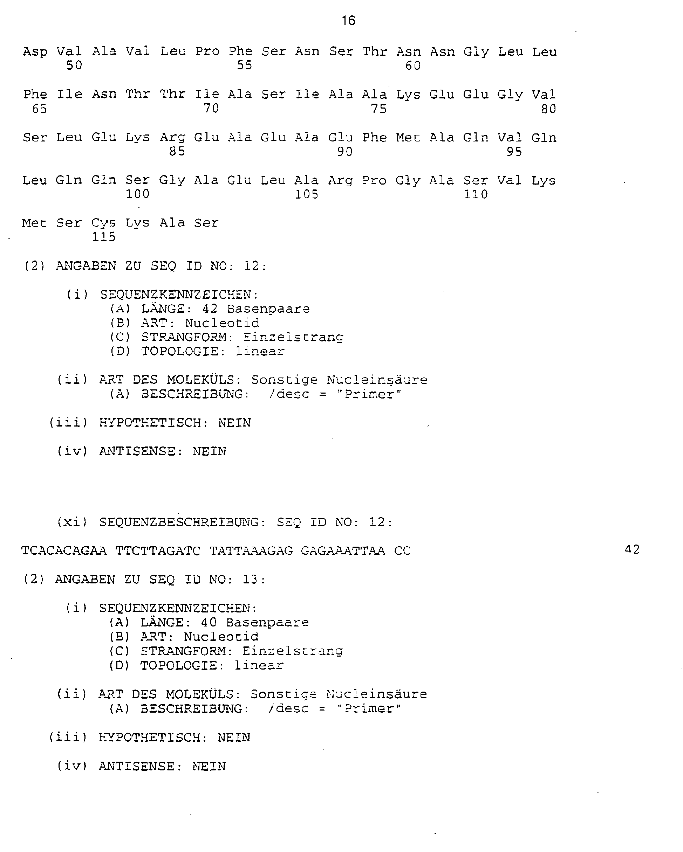

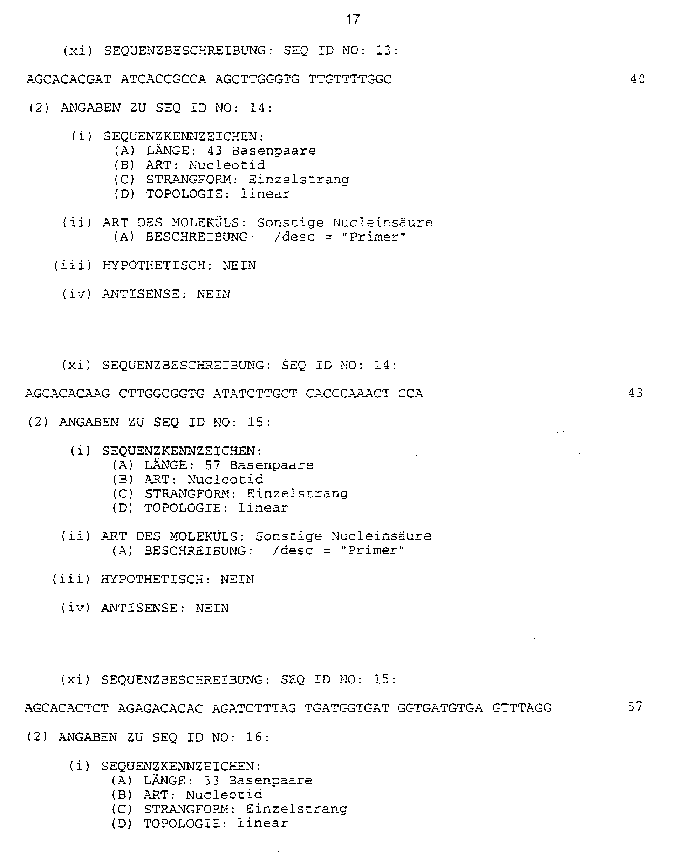

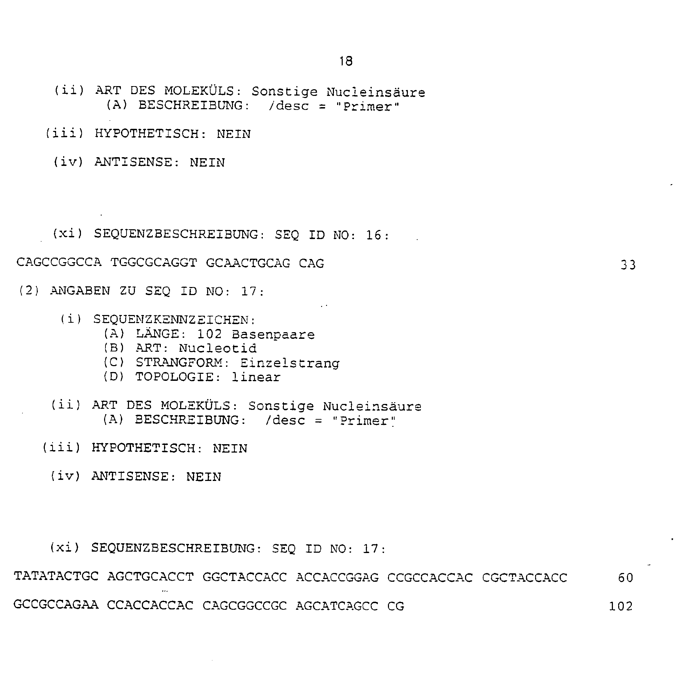

**Fig. 6** zeigt ein Diagramm des Expressionsplasmids pDISC6-SL. 6xHis: Sequenz, die für sechs C-terminale Histidinreste codiert; bla: Gen, das für β-Lactamase kodiert, die für Ampicillinresistenz verantwortlich ist; bp: Basenpaare; *c-myc*: Sequenz, kodierend für ein Epitop, das von dem Antikörper 9E10 erkannt wird; hok-sok: Plasmid-stabilisierender DNA-Locus; Lacl: Gen, das für den Lac-Repressor kodiert; Lac P/O: wt lac-Operon-Promotor/Operator; LacZ': Gen, das für das a-Peptid von β-Galactosidase kodiert; Linker 1: Sequenz, die für ein GlyGly-Dipeptid kodiert, das die V_{H}- und V_{L}-Domänen verknüpft; Linker 3: Sequenz, die für ein GlyGlyProGlySer-Oligopeptid kodiert, das die hybriden scFv-Fragmente verknüpft; M 13 IG: intergenische Region des Bakteriophagen M 13; pBR322ori: Ursprung der DNA-Replikation; Pel-B-Leader: Signalpeptidsequenz der bakteriellen Pectatlyase; rbs: Ribosomenbindungsstelle, die von dem E. coli lacZ Gen (lacZ), von dem Bakteriophagen T7 Gen 10 (T7g10) oder von dem E. coli skp Gen (skp) stammt; skp: Gen, das für den bakteriellen periplasmatischen Faktor Skp/OmpH kodiert; tHP: starker Transkriptions-Terminator; tIPP: Transkriptions-Terminator; V_{H} und V_{L}: variable Region der schweren und der leichten Kette.

Die Erfindung wird durch die nachfolgenden Beispiele erläutert.

### Beispiel 1: Konstruktion des Plasmids pDISC3x19-SL zur Expression von bivalenten, bispezifischen bzw. tetravalenten, bispezifischen Fᵥ-Antikörper-Konstrukten in Bakterien

Die Plasmide pHOG-αCD19 und pHOG-dmOKT3, welche für die scFv-Fragmente kodieren, die von dem Hybridom HD37, das für menschliches CD19 (Kipriyanov et al., 1996, J. Immunol. Meth. 196, 51-62) spezifisch ist, bzw. von dem Hybridom OKT3, das für menschliches CD3 (Kipriyanov et al., 1997, Protein Eng. 10, 445-453) spezifisch ist, abgeleitet sind, wurde zur Konstruktion von Expressionsplasmiden für ein einzelkettiges Fᵥ-Antikörper-Konstrukt verwendet. Ein PCR-Fragment 1 der V_{H}-Domäne von Anti-CD19, gefolgt von einem Segment, das für einen GlyGly-Linker codiert, wurde unter Verwendung der Primer DP1, 5'-TCA-CACAGAATTCTTAGATCTATTAAAGAGGAGAAATTAACC, und DP2, 5'-AGCACACGATATCACCGCCAAGCTTGGGTGTTGTTTTGGC, erzeugt (vgl. Fig. 2). Das PCR-Fragment 1 wurde mit *Eco*RI und *Eco*RV gespalten und mit dem mit *Eco*RI/*Eco*RV linearisierten Plasmid pHOG-dmOKT3 ligiert, wodurch der Vektor pHOG19-3 erzeugt wurde. Das PCR-Fragment 2 der V_{L}-Domäne von Anti-CD19, gefolgt von einem Segment, das für ein c-myc-Epitop und einen Hexahistidinylschwanz codiert, wurde unter Verwendung der Primer DP3, 5'-AGCACA-CAAGCTTGGCGGTGATATCTTGCTCACCCAAACTCCA, und DP4, 5'-AGCA-CACTCTAGAGACACACAGATCTTTAGTGATGGTGATGGTGATGTGAGTTTAGG, erzeugt. Das PCR-Fragment 2 wurde mit *Hind*III und *Xba*l gespalten und mit dem durch *'Hind*III/*Xba*I linearisierten Plasmid pHOG-dmOKT3 ligiert; wodurch der Vektor pHOG3-19 erhalten wurde (vgl. Fig. 2). Das für das hybride scFv-3-19 codierende Gen in dem Plasmid pHOG3-19 wurde mittels PCR mit den Primern Bi3sk, 5'-CAGCCGGCCATGGCGCAGGTGCAACTGCAGCAG und Li-2, 5'-TATA-TACTGCAGCTGCACCTGCGACCCTGGGCCACCAGCGGCCGCAGCATCAGCCCG, zur Erzeugung eines kurzen, starren GGPGS-Linkers (PCR-Fragment 4, vgl. Fig. 2) amplifiziert. Das Expressionsplasmid pDISC3×19-SL wurde durch Ligierung des Ncol/Pvull-Restriktionsfragments aus pHOG19-3, umfassend das Vektorgerüst und das *Nco*I/*Pvu*II-gespaltenen PCR-Fragment 4 konstruiert (vgl. Fig. 3, 4). Die vollständige Nukleotid- und Proteinsequenzen der tetravalenten Fᵥ-Antikörper-Konstrukte sind in der Figur 4. angegeben.

### Beispiel 2: Konstruktion des Plasmids pPIC-DISC-SL zur Expression von tetravalenten, bispezifischen Fᵥ-Antikörper-Konstrukten in Hefe

### Konstruktion von pPIC-DISC-SL

Der Vektor pPICZαA (Invitrogen BV, Leek, Niederlande) zur Expression und Sekretion von rekombinanten Proteinen in der Hefe *Pichia pastoris* wurde als Ausgangsmaterial verwendet. Er enthält ein Gen, das für das *Saccharomyces cerevisiae* α-Faktor-Sekretionssignal codiert, gefolgt von einem Polylinker. Die Sekretion dieses Vektors beruht auf dem dominanten selektierbaren Marker, Zeocin^{™}, der sowohl in *Pichia* als auch in *E. coli* bifunktionell ist. Das Gen, das für das tetravalente Fᵥ-Antikörper-Konstrukt (scDia-SL) codiert, wurde mittels PCR von der Matrize pDISC3x19-SL unter Verwendung der Primer 5-PIC, 5'-CCGTGAAT-TCCAGGTGCAACTGCAGCAGTCTGGGGCTGAACTGGC, und pSEXBn 5'-GGTC-GACGTTAACCGACAAACAACAGATAAAACG amplifiziert. Das so erhaltene PCR-Produkt wurde mit *Eco*RI und *Xba*l gespalten und in mit *EcoR*I/*ba*I linearisiertes pPICZαA ligiert. Es wurde das Expressionsplasmid pPIC-DISC-SL erhalten. Die Nukteotid-und Proteinsequenzen des tetravalenten Fᵥ-Antikörper-Konstruktes sind in Fig. 5 gezeigt.

### Beispiel 3: Expression des tetravalenten Fᵥ-Antikörper-Konstruktes in Bakterien

*E*. *coli*-XL1-Blue-Zellen (Stratagene, La Jolla, CA), die mit dem Expressionsplasmid bzw. pDISC3x19-SL transformiert worden waren, wurden über Nacht in 2xYT-Medium mit 50 µg/ml Ampicillin und 100 mM Glucose (2xYT_{Ga}) bei 37°C gezüchtet. 1:50-Verdünnungen der Übernachtkulturen in 2xYT_{GA} wurden als Kolbenkulturen bei 37°C unter Schütteln mit 200 UpM gezüchtet. Als die Kulturen einen OD₆₀₀-Wert von 0,8 erreicht hatten, wurden die Bakterien durch 10minütige Zentrifugation mit 1500 g bei 20°C pelletiert und in über Nacht in 2xYT-Medium mit 50 *µ*g/ml Ampicillin und 100 mM Glucose (2xYT_{Ga}) bei 37°C gezüchtet. 1:50-Verdünnungen der Übernachtkulturen in 2xYT_{GA} wurden als Kolbenkulturen bei 37°C unter Schütteln mit 200 UpM gezüchtet. Als die Kulturen einen OD₆₀₀-Wert von 0,8 erreicht hatten, wurden die Bakterien durch 10minütige Zentrifugation mit 1500 g bei 20°C pelletiert und in dem gleichen Volumen eines frischen 2xYT-Mediums, das 50 *µ*g/ml Ampicillin und 0,4 M Saccharose enthielt, resuspendiert. IPTG wurde bis zu einer Endkonzentration von 0,1 mM zugesetzt, und das Wachstum wurde bei Raumtemperatur (20-22°C) 18-20 h fortgesetzt. Die Zellen wurden durch 10minütige Zentrifugation mit 5000 g bei 4°C geerntet. Der Kulturüberstand wurde zurückgehalten und auf Eis gelagert. Um die löslichen periplasmatischen Proteine zu isolieren, wurden die pelletierten Bakterien in 5% des Anfangsvolumens an eiskalter 50 mM Tris-HCl, 20% Saccharose, 1 mM EDTA, pH 8,0, resuspendiert. Nach einer 1 stündigen Inkubation auf Eis unter gelegentlichem Rühren wurden die Sphäroplasten mit 30.000 g 30 min bei 4°C zentrifugiert, wobei der lösliche periplasmatische Extrakt als Überstand und die Sphäroplasten mit dem unlöslichen periplasmatischen Material als Pellet erhalten wurden. Der Kulturüberstand und der lösliche periplasmatische Extrakt wurden vereinigt, durch weitere Zentrifugation (30.000 g, 4°C, 40 min) geklärt. Das rekombinante Produkt wurde durch Ammoniumsulfatfällung (Endkonzentration 70% Sättigung) eingeengt. Das Proteinpräzipitat wurde durch Zentrifugation (10.000 g, 4°C, 40 min) gewonnen und in 10% des Anfangsvolumens an 50 mM Tris-HCl, 1 M NaCl, pH 7,0, aufgelöst. Eine immobilisierte Metallaffinitätschromatographie (IMAC) wurde bei 4°C unter Verwendung einer 5 ml Säule an chelatierender Sepharose (Pharmacia), die mit Cu²⁺ beladen war und mit 50 mM Tris-HCl, 1 M NaCl, pH 7,0 (Startpuffer) equilibriert worden war, durchgeführt. Die Probe wurde durch ihr Leiten über die Säule aufgeladen. Sie wurde dann mit zwanzig Säulenvolumina Startpuffer, gefolgt von Startpuffer mit 50 mM Imidazol, bis die Absorption bei 280 nm des Effluenten minimal war, gewaschen (etwa dreißig Säulenvolumina). Das absorbierte Material wurde mit 50 mM Tris-HCl, 1 M NaCl, 250 mM Imidazol, pH 7,0, eluiert.

Die Proteinkonzentrationen wurden mit dem Bradford-Farbstoffbindungstest (1976,

### Beispiel 4: Expression des tetravalenten Antikörper-Konstruktes in der Hefe Pichia pastoris

Kompetente *P. pastoris* GS 155-Zellen (Invitrogen) wurden in Gegenwart von 10 µg Plasmid-DNA von pPIC-DISC-SL, die mit *Sac*I linearisiert worden war, elektroporiert. Die Transformanten wurden 3 Tage bei 30°C auf YPD-Platten, die 100 µg/ml Zeocin^{™} enthielten, selektiert. Die Klone, die bivalente bzw. tetravalente Fᵥ-Antikörper-Konstrukte sezernierten, wurden durch Plattenscreening unter Verwendung eines anti-c-*myc*-mAk 9E10 (IC Chemikalien, Ismaning, Deutschland) selektiert.

Zur Expression der tetravalenten Fᵥ-Antikörper-Konstrukte wurden die Klone in YPD-Medium in Schüttelkolben 2 Tage bei 30°C unter Rühren gezüchtet. Die Zellen wurden zentrifugiert, in dem gleichen Volumen des Mediums, das Methanol enthielt, resuspendiert und weitere 3 Tage bei 30°C unter Rühren inkubiert. Die Überstände wurden nach der Zentrifugation gewonnen. Das rekombinante Produkt wurde durch Ammoniumsulfatfällung, gefolgt von IMAC, wie vorstehend beschrieben, isoliert.

### Beispiel 5: Charakterisierung des tetravalenten Fᵥ-Antikörper-Konstruktes

### (A) Größenausschlußchromatographie

Eine analytische Gelfiltration der Fᵥ-Antikörper-Konstrukte wurde in PBS unter Verwendung einer Superdex-200-HR10/30-Säule (Pharmacia) durchgeführt. Das Probenvolumen und die Fließgeschwindigkeit betrugen 200 µl/min bzw. 0,5 ml/min. Die Säule wurde mit hoch- und niedermolekularen Gelfiltrations-Kalibrationskits (Pharmacia) kalibriert.

### (B) Durchflußzytometrie

Probenvolumen und die Fließgeschwindigkeit betrugen 200 µl/min bzw. 0,5 ml/min. Die Säule wurde mit hoch- und niedermolekularen Gelfiltrations-Kalibrationskits (Pharmacia) kalibriert.

### (B) Durchflußzytometrie

Die menschliche CD3⁺/CD19-akute-T-Zell-Leukämielinie Jurkat und die CD19⁺/CD3-B-Zellinie JOK-1 wurden für die Durchflußzytometrie verwendet. 5 x 10⁵ Zellen in 50 µl RPMI 1640-Medium (GIBCO BRL, Eggestein, Deutschland), das mit 10% FCS und 0,1% Natriumazid supplementiert war (als vollständiges Medium bezeichnet), wurden mit 100 µl der Fᵥ-Antikörper-Präparate 45 min auf Eis inkubiert. Nach Waschen mit dem vollständigen Medium wurden die Zellen mit 100 µl 10 µg/ml anti-c-*myc*-Mak 9E10 (IC Chemikalien) in dem gleichen Puffer 45 min auf Eis inkubiert. Nach einem zweiten Waschzyklus wurden die Zellen mit 100 µl des FITCmarkierten Ziege-anti-Maus-IgG (GIBCO BRL) unter den gleichen Bedingungen wie vorher inkubiert. Die Zellen wurden dann erneut gewaschen und in 100 µl 1 µg/ml-Propidiumiodid-Lösung (Sigma, Deisenhofen, Deutschland) in vollständigem Medium unter Ausschluß von toten Zellen resuspendiert. Die relative Fluoreszens der gefärbten Zellen wurde unter Verwendung eines FACScan-Durchflußzytometers (Becton Dickinson, Mountain View, CA) gemessen:

### (C) Cytotoxizitätstest

Die CD19-exprimierende Burkitt-Lymphoma-Zellinie Raji und Namalwa wurden als Zielzellen verwendet. Die Zellen wurden in RPMI 1640 (GIBCO BRL), das mit 10% hitzeinaktiviertem FCS (GIBCO BRL), 2 mM Glutamin und 1 mM Pyruvat supplementiert war, bei 37°C in einer befeuchteten Atmosphäre mit 7,5% CO₂ inkubiert. Die cytotoxischen T-Zell-Tests wurden in RPMI-1640-Medium, das mit 10% FCS, 10 mM HEPES, 2 mM Glutamin, 1 mM Pyruvat und 0,05 mM 2-ME supplementiert war, durchgeführt. Die cytotoxische Aktivität wurde unter Verwendung eines Standard[⁵¹Cr]-Freisetzungstests bewertet; 2 x 10⁶ Zielzellen wurden mit 200 µCi zugesetzt. Der gesamte Test wurde dreifach angesetzt und 4 h bei 37°C inkubiert. 100 µl des Überstands wurden gewonnen und auf [⁵¹Cr]-Freisetzung in einem gamma-Zähler (Cobra Auto Gamma; Canberra Packard, Dreieich, Deutschland) getestet. Die maximale Freisetzung wurde durch Inkubation der Zielzellen in 10% SDS bestimmt, und die spontane Freisetzung wurde durch Inkubation der Zellen in Medium allein bestimmt. Die spezifische Lyse (%) wurde berechnet als: (experimentelle Freisetzung - spontane Freisetzung)/(maximale Freisetzung - spontane Freisetzung) x 100.

### Beispiel 6: Konstruktion des Plasmids pDISC6-SL zur Expression von tetravalenten, bispezifischen Fᵥ-Antikörper-Konstrukten in Bakterien durch Hoch-Zelldichte-Fermentation

Es wurden Expressionsvektoren hergestellt, die das hok/sok Plasmid-freie Zell-"suicide"-System und ein Gen enthielten, das für den Skp/OmpH periplasmatischen Faktor für eine größere Herstellung rekombinanter Antikörper kodiert. Das skp Gen wurde durch PCR mittels der Primer skp-1, 5'-CGA ATT CTT AAG ATA AGA AGG AGT TTA TTG TGA AAA AGT GGT TAT TAG CTG CAG G und skp-2, 5'-CGA ATT AAG CTT CAT TAT TTA ACC TGT TTC AGT ACG TCG G unter Verwendung des Plasmids pGAH317 (Holck and Kleppe, 1988, Gene 67, 117-124) amplifiziert. Das erhaltene PCR-Fragment wurde mit AfIII und HindIII gespalten und in das mit AfIII/HindIII linearisierte Plasmid pHKK (Horn et al., 1996, Appl. Microbiol. Biotechnol. 46, 524-532) inseriert, wodurch der Vektor pSKK erhalten wurde. Die im Plasmid pDISC3x19-SL enthaltenen und für die scFv-Antikörper-Konstrukte kodierenden Gene wurden durch PCR mittels der Primer fe-1, 5'-CGA ATT TCT AGA TAA GAA GGA GAA ATT AAC CAT GAA ATA CC und fe-2, 5'-CGA ATT CTT AAG CTA TTA GTG ATG GTG ATG GTG ATG TGA G amplifiziert. Die Xbal/Aflll gespaltenen PCR-Fragmente wurden in pSKK vor dem skp Insert inseriert, wodurch das Expressionsplasmid pDISC6-SL erhalten wurde, das ein tri-cistronisches Operon unter der Kontrolle des lac Promotor/Operator-Systems enthält (vgl. Fig. 6).

## Patentansprüche

1. Tetravalenter Fᵥ-Antikörper-Dimer, wobei ein einzelkettiges Fᵥ-Antikörper-Konstrukt mit einem anderen, gleichartigen, einzelkettigen Fᵥ-Antikörper-Konstrukt zusammengefaltet ist und die beiden einzelkettigen Fᵥ-Antikörper-Konstrukte jeweils vier variable Domänen umfassen, die über einen Peptidlinker 1, einen mittleren Peptidlinker 2 und einen Peptidlinker 3 miteinander verbunden sind, wobei die Peptidlinker 1 und 3 0-10 Aminosäuren aufweisen.

2. Fᵥ-Antikörper-Dimer nach Anspruch 1, wobei der mittlere Peptidlinker 23-10 Aminosäuren aufweist.

3. Fᵥ-Antikörper-Dimer nach Anspruch 1 oder 2, wobei der Fᵥ-Antikörper-Dimer bispezifisch ist.

4. Fᵥ-Antikörper-Dimer nach Anspruch 3, wobei der Fᵥ-Antikörper-Dimer spezifisch für CD3 und CD19 ist.

5. Fᵥ-Antikörper-Dimer nach Anspruch 1 oder 2, wobei der Fᵥ-Antikörper-Dimer monospezifisch ist.

6. Verfahren zur Herstellung des tetravalenten Fᵥ-Antikörper-Dimer nach einem der Ansprüche 1- 5, wobei für die Peptidlinker 1, 2 und 3 kodierende DNAs mit für die vier variablen Domänen eines einzelkettigen Fᵥ-Antikörper-Konstruktes kodierenden DNAs ligiert werden derart, daß die Peptidlinker die variablen Domänen miteinander verbinden, und das erhaltene DNA-Molekül in einem Expressionsplasmid exprimiert wird.

7. Expressionsplasmid, kodierend für das einzelkettige Fᵥ-Antikörper-Konstrukt nach einem der Ansprüche 1- 5.

8. Expressionsplasmid nach Anspruch 7, nämlich pDISC3x19-SL, hinterlegt unter der Nummer DSM 12149.

9. Expressionsplasmid nach Anspruch 7, nämlich pPIC-DISC-SL, hinterlegt unter der Nummer DSM 12151.

10. Expressionsplasmid nach Anspruch 7, nämlich pDISC6-SL, wie in Figur 6 definiert.

11. Verwendung des tetravalenten Fᵥ-Antikörper-Dimers nach einem der Ansprüche 1- 5 zur Herstellung einer diagnostischen oder therapeutischen Zusammensetzung für die Diagnose und/oder Therapie von Erkrankungen.

12. Verwendung nach Anspruch 11, wobei die Erkrankungen virale, bakterielle oder Tumor-Erkrankungen sind.

## Claims

1. A tetravalent Fᵥ antibody dimer, wherein a monomeric Fᵥ antibody construct is folded together with another monomeric Fᵥ antibody construct of the same kind and each of both monomeric Fᵥ antibody constructs comprises four variable domains linked with one another via the peptide linker 1, a middle peptide linker 2 and a peptide linker 3, wherein the peptide linkers 1 and 3 have 0-10 amino acids.

2. The Fᵥ antibody dimer according to claim 1, wherein the middle peptide linker 2 has 10 amino acids.

3. The Fᵥ antibody dimer according to claim 1 or 2, wherein the Fᵥ antibody dimer is bispecific.

4. The Fᵥ antibody dimer according to claim 3, wherein the Fᵥ antibody dimer is specific for CD3 and CD19.

5. The Fᵥ antibody dimer according to claim 1 or 2, wherein the Fᵥ antibody dimer is monospecific.

6. A method of producing the tetravalent Fᵥ antibody dimer according to any of claims 1-5, wherein DNAs coding for the peptide linkers 1, 2 and 3 are ligated with DNAs coding for the four variable domains of a monomeric Fᵥ antibody construct such that the peptide linkers link the variable domains with one another and the resulting DNA molecule is expressed in an expression plasmid.

7. An expression plasmid coding for the monomeric Fᵥ antibody construct according to any of claims 1-5.

8. The expression plasmid according to claim 7, namely pDISC3x19-SL deposited under the number DSM 12149.

9. The expression plasmid according to claim 7, namely pPIC-DISC-SL deposited under the number DSM 12151.

10. The expression plasmid according to claim 7, namely pDISC6-SL as defined in Fig. 6.

11. A use of the tetravalent Fᵥ antibody dimer according to any of the claims 1-5 for the preparation of a diagnostic or pharmaceutical composition for the diagnosis and/or treatment of diseases.

12. The use according to claim 11, wherein the diseases are viral, bacterial or tumoral diseases.

## Revendications

1. Dimère anticorps Fv tétravalent dans lequel une construction anticorps Fv à chaîne unique est précipitée avec une autre construction à chaîne unique de même sorte et les deux constructions anticorps Fv à chaîne unique englobent quatre domaines variables, qui sont liées par un agent de liaison peptide 1, un agent de liaison peptide médian 2 et un agent de liaison peptide 3, où les agents de liaison peptide 1 et 3 comprennent de 0-10 acides aminés.

2. Dimère anticorps Fv selon la revendication 1, dans lequel l'agent de liaison peptide médian 3 comprend 3 à 10 acides aminés.

3. Dimère anticorps Fv selon la revendication 1 ou 2, dans lequel le dimère anticorps Fv est bi-spécifique.

4. Dimère anticorps Fv selon la revendication 3, dans lequel le dimère anticorps Fv est spécifique pour CD3 et CD19.

5. Dimère anticorps Fv selon la revendication 1 ou 2, dans lequel le dimère anticorps Fv est mono-spécifique.

6. Procédé de préparation du dimère anticorps Fv tétravalent selon l'une des revendications 1 à 5,
**caractérisé en ce que** les ADN codant pour les agents de liaison peptide 1, 2 et 3 sont ligaturés de telle manière avec les ADN codant pour les quatre domaines variables d'une construction d'anticorps Fv à chaîne unique que les agents de liaison peptide qui réunissent les domaines variables ensemble et la molécule ADN est exprimée en un plasmide d'expression.

7. Plasmide d'expression pour la construction d'anticorps Fv à chaîne unique selon l'une des revendications 1 à 5.

8. Plasmide d'expression selon la revendication 7, à savoir pDISC3x19-SL où il est déposé sous le numéro DSM12149.

9. Plasmide d'expression selon la revendication 7, à savoir pPIC-DISC-SL où il est déposé sous le numéro DSM12151.

10. Plasmide d'expression selon la revendication 7, à savoir pDISC6-SL, est défini à la figure 6.

11. Utilisation du dimère anticorps Fv tétravalent selon l'une des revendications 1 à 5 à la préparation d'une composition de diagnostique ou thérapeutique pour le diagnostique et/ou le traitement des maladies.

12. Utilisation selon la revendication 11, dans laquelle les maladies sont virales, bactériennes ou tumorales.
